# EUROPEAN PATENT APPLICATION

(11) **EP 2 594 261 A1**
(43) Date of publication of application: **22.05.2013**
(21) Application number: 11189724.5
(22) Date of filing: 18.11.2011
(51) Int. Cl.: A61K 9/70, A61K 31/27, A61P 25/28

(54) **Composition for transdermal administration of rivastigmine**

(71) Applicant: Labtec GmbH, 40764 Langenfeld (DE)
(72) Inventor: Breitenbach, Armin, 51371 Leverkusen (DE); Braun, Sebastian, Wermelskirchen (DE); Becker, Ulrich, 40764 Langenfeld (DE)
(74) Representative: von Renesse, Dorothea

(57) **Abstract**

It has been found that phenyl carbamates, in particular Rivastigmine, can be formulated as a stable TTS by using a monolayer poly alkyl (meth)acrylates, wherein the poly alkyl (meth)acrylates are substantially free of free carboxylate groups.

## Description

The present invention relates to compositions for transdermal administration of phenyl carbamates, in particular to pharmaceutical compositions for transdermal administration of (*S*)-{3-[α-(Dimethylamino)ethyl]phenyl}-N-ethyl-N-methylcarbamat (Rivastigmine).

Rivastigmine base is an active pharmaceutical ingredient (API) for Alzheimer's disease and Parkinson related dementia. Dementia of Alzheimer's disease is correlated with the reduced formation of acetylcholine due to a reduced activity of the choline-acetyltransferase. In addition, acetylcholine degradation is increased due to the enhanced expression of the butyrylcholin esterase which also cleaves acetylcholine. Rivastigmine base is an irreversible cholinesterase inhibitor, increasing the total amount of acetylcholine by inhibiting actylcholine esterases and butyrylcholine esterases preventing the fast degradation of acetylcholine.

Due to the low bioavailability and half-life of Rivastigmine, transdermal therapeutic systems (TTS) were developed and are disclosed in GB 2203040 A and EP 1047409 A1 as suitable systems for the administration of the drug. In both documents the use of Rivastigmine in combination with acrylate polymers is disclosed.

In EP 1047409 it is described that the compositions according to GB 2203040 degrade, possibly due to oxidative degradation, despite the formation of an occlusive polymer matrix around Rivastigmine and its storage in air-tight packaging. According to EP 1047409, the problem of degradation of Rivastigmine is overcome by formulation of Rivastigmine in combination with an antioxidant.

From W02011/076621 A2 a multilayer transdermal therapeutic system with a reservoir layer comprising Rivastigmine is known. The problem of degradation of Rivastigmine is overcome by using polymers which are free of carboxyl and hydroxyl groups. A separate adhesive layer is used to achieve sufficient adhesiveness of the TTS. However, multilayer systems are disadvantageous in particular with regards to the intense skin contact and skin delivery as well as in terms of manufacturing costs.

From EP 2 172 194 A1, a monolithic transdermal therapeutic system with Rivastigmine is known. The problem of a reduced cohesiveness of polymeric adhesives at the presence of Rivastigmine is particularily addressed. In order to overcome this problem it is suggested to use acrylic polymers with a moderate shear. Furthermore it is suggested that the acrylic polymers contain hydroxyl and carboxyl groups in order to reduce skin irritation and to control drug delivery. The addition of diffusible adjuvants like squalene or trietylcitrate is suggested as well.

KR20100080681 discloses a two-layered Rivastigmine containing TTS which provides enforced delivery by adding acids, specifically diffusive carboxyl acids to the formulation. This technique is likely to irritate the skin unnecessarily and shares the disadvantages of a two-layered composition.

The objective of the present invention thus is to provide a transdermal therapeutic system (TTS) with phenylcarbamates, in particular Rivastigmine, which are simple to manufacture and, at the same time, allow sufficient stability. In preferred embodiments the TTS of the invention furthermore enables a high degree of delivery rate of the drug substance. The TTS of the invention favourably compensates the softenising properties of the drug substance or softenizing excipients.

It was found by the inventors, surprisingly, that the critical impact of Rivastigmine on the cohesiveness of acrylic polymer adhesives is compensated when a mixture of at least one poly alkyl acrylate and at least one poly alkyl methacrylate is applied. If these polymers are substantially free of free carboxylates, in particular both polymer- bound and small molecular carboxylates, Rivastigmine is sufficiently stable.

Hence according to the invention, it is possible to provide a TTS comprising Rivastigmine, which does not require at least two separate layers (namely one reservoir containing the drug substance and a second layer with the adhesive), but comprises a single layer with the adhesive and the drug substance (mono layer). Thus in one aspect, the invention provides a monolithic TTS with Rivastigmine.

It was further surprising that the TTS of the invention does not only exhibit excellent stability, but preferably shows also improved pharmacokinetic properties as compared to Rivastigimine currently available on the market. This allows reducing the overall size of the TTS compared to present market products.

In a preferred embodiment, within the mono layer poly alkyl acrylate and poly alkyl methacrylate form a biphasic system. This system contains the poly alkyl methacrylic polymer as the inner phase and the poly alkyl acrylate as the continuous phase. The inventors have found that this biphasic system is particularily advantageous for maintaining the cohesiveness of the acrylic adhesives even at the presence of Rivastigmine.

In a yet another particularily preferred embodiment of the invention the poly alky methacrylate comprises aminic groups. It is believed - without being bound to this theory - that the presence of these aminic groups increases the drug flux. This is advantageous since it allows reducing the drug load in the adhesive without at the same time reducing the drug flux. Hence with less drug substance the same drug flux can be maintained. Furthermore it is possible to even increase the drug flux with higher drug substance concentration.

The term "poly (meth)acrylate" means according to the invention both, poly acrylate as well as poly methacrylate, unless otherwise specifically outlined.

"Monolayer TTS" as used in the present context means a TTS with a single adhesive layer of the TTS comprising the drug substance. The adhesive layer with the drug substance is called "monolayer".Decisive for the TTS according to this invention is the absence of a second or more layer with adhesives. This system sometimes is called "monolithic TTS".

Within the monolayer the polymers may form - due to their solubility properties - separate phases, which may be microscopically visible and appear as droplets of the inner phase of typically 5-100 µm. At least a part of of Rivastigmine can be taken up in the dispersed phase.

"Substantially free of free carboxylate groups" means according to the invention that less than 3 % of the monomers of the polymer, more preferably less than 2 % of the monomers of the polymer, in particular less than 1 % of the monomers of the polymer comprise a free carboxylate group, wherein "free carboxylate group" means the hydrogenated carboxylate group (carboxylic acid) or a salt thereof.

In a preferred embodiment of the invention the poly alkyl (meth)acrylate, in particular the poly alkyl acrylate and/or the poly alkyl methacrylate, is characterized by comprising no free carboxylate group, at all.

The term "poly alkyl acrylate" means according to the invention a homopolymer or copolymer which comprises alkyl acrylate monomers, wherein the ratio of the number of alkyl acrylate monomers to the number of alkyl methacrylate monomers is at least 4:1, more preferably at least 8:1, in particular at least 12:1. In a preferred embodiment the "poly alkyl acrylate" does not contain alkyl methacrylate monomers, at all. In the poly alkyl acrylate copolymer preferably at least 40 %, more preferably at least 60 %, in particular at least 80 % of the monomers are alkyl acrylate monomers.

Vice versa the term "poly alkyl methacrylate" means according to the invention a homopolymer or copolymer which comprises alkyl methacrylate monomers, wherein the ratio of the number of alkyl methacrylate monomers to the number of alkyl acrylate monomers is at least 4:1, more preferably at least 8:1, in particular at least 12:1. In a preferred embodiment the "poly alkyl methacrylate" does not contain alkyl acrylate monomers, at all. In the poly alkyl methacrylate copolymer preferably at least 40 %, more preferably at least 60 %, in particular at least 80 % of the monomers are alkyl methacrylate monomers.

The preferred phenyl carbamate according to the invention is Rivastigmine ((S)-N-ethyl-3-[(1-dimethylamino)ethyl]-N-methyl-phenyl-carbamate or, according to IUPAC, (*S*)-{3-[α-(Dimethylamino)ethyl]phenyl}-N-ethyl-N-methylcarbamate) or salts thereof. A preferred salt of Rivastigmine is tartrate.

The "neutralization" of free carboxylate groups is preferably realized by formation of (carboxylic acid) ester groups. Thus the poly alkyl (meth)acrylate is characterized by comprising (carboxylic acid) ester groups, preferably C₁₋₁₀ alkyl ester groups, more preferably C₁₋₆ alkyl ester groups, wherein preferably all of the neutralized carboxylate groups are in the form of ester groups.

In another preferred embodiment of the invention the poly alkyl (meth)acrylate, in particular the poly alkyl acrylate and/or the poly alkyl methacrylate, is characterized by comprising no functional groups, at all. That means that all potentially functional groups like carboxylate groups, carboxamide groups or hydroxyl groups are neutralized by defunctionalisation, for example by formation of alkyl ester, (di)alkylamido or ether groups, resulting in a polymer which does not comprise reactive groups, at all.

But in another preferred embodiment of the invention, the poly alkyl (meth)acrylate, in particular the poly alkyl acrylate and/or the poly alkyl methacrylate, which is substantially or totally free of free carboxylate groups, does comprise other functional groups, in particular amino groups and/or hydroxyl groups.

In a preferred embodiment of the invention the amino groups and/or hydroxyl groups are bound to alkyl residues of the alkyl carboxy ester groups of the poly alkyl (meth)acrylate, such resulting in heterofunctionalized alkyl residues. In a preferred embodiment the number of heterofunctionalized alkyl residues, if present, is from 5 to 70 %, more preferably from 10 to 60 %, of the total number of alkyl ester residues.

In one preferred embodiment the poly alkyl (meth)acrylate, in particular the poly alkyl acrylate and/or the poly alkyl methacrylate, preferably the poly alkyl acrylate, contains hydroxy alkyl, preferably C₁₋₁₀ hydroxy alkyl, more preferably C₁₋₆ hydroxy alkyl ester groups.

In another preferred embodiment the poly alkyl (meth)acrylate, in particular the poly alkyl acrylate and/or the poly alkyl methacrylate, preferably the poly alkyl methacrylate, contains heteroalkyl ester groups, preferably C₁₋₁₀ heteroalkyl, more preferably C₁₋₆ heteroalkyl groups, wherein the heteroatom is preferably nitrogen, in particular in the form of a tertiary amino group.

In one preferred embodiment of the invention as the poly alkyl (meth)acrylate a poly alkyl acrylate is used. In another preferred embodiment of the invention as the poly alkyl (meth)acrylate a poly alkyl methacrylate is used.

In a particularly preferred embodiment of this very preferred embodiment the mono layer of the TTS according to the invention comprises
a) 1 to 60 wt.-% of a phenyl carbamate, in particular Rivastigmine;
b) 5 to 70 wt.-% of at least one poly alkyl acrylate as hereinbefore defined; and
c) 5 to 70 wt.-% of at least one poly alkyl methacrylate as hereinbefore defined.

In a even more preferred embodiment of this very preferred embodiment the mono layer according to the invention comprises
a) 10 to 50 wt.-%, in particular 20 to 40 wt.-%, of a phenyl carbamate, in particular Rivastigmine;
b) 20 to 60 wt.-%, in particular 30 to 50 wt.-%, of at least one poly alkyl acrylate as hereinbefore defined; and
c) 10 to 50 wt.-%, in particular 20 to 40 wt.-%, of at least one poly alkyl methacrylate as hereinbefore defined.

In a most preferred embodiment the mono layer contains phenyl carbamate (e.g. Rivastigmine) in 30% wt.-%, pol acrylate in 40 % wt.- and 30 % wt.-% poly alkyl methacrylate.

The molecular weight of the poly alkyl (meth)acrylate, in particular of the poly alkyl acrylate and/or poly alkyl methacrylate, is preferably between 10,000 and 300,000 g/mol, more preferably between 20,000 and 250,000 g/mol, in particular between 30,000 and 200,000 g/mol.

In one preferred embodiment of the invention the poly alkyl acrylate according to the invention is a homopolymer of alkyl acrylate monomers. Such preferred homopolymers of alkyl acrylate monomers which contain alkyl ester groups and are free of free carboxylate groups are for example available under the tradename DURO-TAK by Henkel (Germany). Examples of suitable polymers are DURO-TAK 387-2610 and DURO-TAK 87-202A (which contain free hydroxyl groups).

In another preferred embodiment of the invention the poly alkyl acrylate according to the invention is a copolymer of alkyl acrylate monomers and other non-ionic monomers, in particular of alkyl acrylate monomers and vinyl acetate monomers. Preferred copolymers of alkyl acrylate monomers and vinyl acetate which contain alkyl ester groups and are free of free carboxylate groups are also available under the tradename DURO-TAK by Henkel (Germany). Examples of suitable polymers are DURO-TAK 387-2287 and 87-4287 (which contain free hydroxyl groups) and a very preferred example is DURO-TAK 87-4098 (which is free of any functional group). The amount of vinyl acetate monomers in this embodiment is preferably from 2 to 40 % of the total number of monomers.

In a very preferred embodiment of the invention the poly alkyl acrylate according to the invention is a copolymer of alkyl acrylate monomers and nitrogen-containing non-ionic monomers, wherein all carboxyl groups are esterified and wherein the nitrogen-containing non-ionic monomers are selected from N-substituted acrylamide monomers, N-substituted methacrylamide monomers, vinylacetamides, nitriles and mixtures thereof. The copolymer preferably comprises 50-98 % of alkyl acrylate monomers and 2-50 % of nitrogen containing non-ionic monomers. The alkyl acrylate is preferably a C₁₋₁₀ alkyl acrylate, more preferably 2-ethylhexyl acrylate and/or n-butyl acrylate, the nitrile is preferably methacrylonitrile or 2-cyanoethylacrylate and the acrylamide is preferably a C₁₋₁₀ acrylamide, more preferably t-octyl acrylamide. A preferred example of this embodiment is DURO-TAK 87-9301 which is free of any functional group.

The poly alkyl methacrylate according to the invention is in a preferred embodiment a homopolymer of alkyl methacrylate monomers, wherein all carboxyl groups are esterified, preferably with C₁₋₈ alkanols and/or with C₁₋₈ heteroalkanols. Preferred homopolymers in this sense which are free of free carboxylate groups are for example available under the tradename EUDRAGIT E PO by Evonik (Germany) (which contains tertiary amino groups) and under the tradename PLASTOID B, also by Evonik (Germany), (which contains no functional group at all).

In a particular preferred embodiment the poly methacrylate is composed of at least 0,5% (wt/wt) dimethylaminoethyl methacrylate.

Preferably the poly alkyl methacrylate of this embodiment is free of any functional group and contains butyl ester groups and methyl ester groups, preferably 60-90 %, in particular 70-80 %, butyl ester groups and 10-40 %, in particular 20-30 % methyl ester groups and has preferably a molecular weight of 120,000 to 180,000 g/mol or the poly alkyl methacrylate comprises tertiary amino groups and contains besides butyl ester groups and methyl ester groups 2-dimethylaminoethyl ester groups, preferably 30-70 %, in particular 40-60 %, 2-dimethylaminoethyl ester groups, 10-40 %, in particular 20-30 %, butyl ester groups and 10-40 %, in particular 20-30 %, methyl ester groups and has preferably a molecular weight of 25,000 to 65,000 g/mol.

In a preferred embodiment of the invention the poly alkyl (meth)acrylate is a graft polymer.

In the most preferred embodiment of the invention a combination of a poly alkyl acrylate which is free of any functional group and a poly alkyl methacrylate which is either also free of any functional group or which contains tertiary amino groups is used.

Very preferred in this sense is a mono layer comprising
a) 1 to 60 wt.-%, in particular 10 to 50 wt.-%, more preferably 20 to 40 wt.-%, of a phenyl carbamate, in particular Rivastigmine;
b) 5 to 70 wt.-%, in particular 20 to 60 wt.-%, more preferably 30 to 50 wt.-%, of at least one poly alkyl acrylate which is free of any functional group; and
c) 5 to 70 wt.-%, in particular 10 to 50 wt.-%, more preferably 20 to 40 wt.-%, of at least one poly alkyl methacrylate which is also free of any functional group.

Another very preferred embodiment in this sense is a mono layer comprising
a) 1 to 60 wt.-%, in particular 10 to 50 wt.-%, more preferably 20 to 40 wt.-%, of a phenyl carbamate, in particular Rivastigmine;
b) 5 to 70 wt.-%, in particular 20 to 60 wt.-%, more preferably 30 to 50 wt.-%, of at least one poly alkyl acrylate which is free of any functional group; and
c) 5 to 70 wt.-%, in particular 10 to 50 wt.-%, more preferably 20 to 40 wt.-%, of at least one poly alkyl methacrylate which is free of functional groups besides tertiary amino groups.

In these most preferred embodiments preferably the preferred poly alkyl acrylates and poly alkyl methacrylates as described before are used, wherein the combination of Rivastigmine, DURO-TAK 87-9301 and EUDRAGIT E PO and the combination of Rivastigmine, DURO-TAK 87-9301 and PLASTOID B are particularly preferred.

For the preparation of the mono layer a suitable solvent is used, preferably ethyl acetate.

In one preferred embodiment of the invention, the TTS according to the invention does not contain any antioxidant.

In another preferred embodiment of the invention, the composition according to the invention contains at least one antioxidant, preferably selected from tocopherol, in particular alpha-tocopherol, and esters thereof, e.g. tocopherol acetate, ascorbic acid and esters thereof, e.g. ascorbyl palmitate, butylhydroxytoluene (BHT), butylhydroxyanisole (BHA), propyl gallate, citraconic acid, glutathione, cysteine, citric acid and beta-carotin.

The antioxidant is, if present, preferably used in an amount of 0.01 to 2 wt.-%, more preferably in an amount of 0.05 to 1 wt.-%, in particular in an amount of 0.05 to 0.5 wt.-%.

In a preferred embodiment of the invention the mono layer is characterized by containing no poly alkyl (meth)acrylate, preferably no polymer at all, more preferably no compound at all, with a free carboxylate group.

In another preferred embodiment of the invention the mono layer is characterized by containing no poly alkyl (meth)acrylate, preferably no polymer at all, more preferably no compound at all, with a functional group. That means all potentially functional groups are defunctionalized, for example by formation of ester or ether bonds.

The mono layer according to the invention may contain further additives, such as plastisizers, softeners or surfactants. Examples for such further additives are polyoxyethylene fatty alcohol ethers, wherein the alcohol is preferably a C₁₂₋₁₈ alcohol, polyoxyethylene sorbitan fatty acid esters, wherein the fatty acid is preferably a C₁₂₋₁₈ fatty acid, polyoxyethylene-(5-40) stearic acid esters, polyoxyethylene glycol fatty alcohol ethers, e.g. polyethylene glycol-(6-25) cetyl ether, glycerin polyethylene ricinoleate or glycerin polyethylene glycol stearate, polyoxyethylene glycols of MW from 200 to 600 Daltons, esters of poly(2-7)ethylene glycol glycerol ether having at least one hydroxyl group and an aliphatic (C₆₋₂₂) carboxylic acid, adipic acid lower alkyl esters, e.g. di-n-butyl adipate and diisopropyl adipate, glycerin polyethylene glycol ricinoleate, e.g. product of 35 moles ethylene oxide and castor oil, triacetin-(1,2,3), fatty acids, preferably C₁₂₋₁₈ fatty acids and fatty alcohols, preferably C₁₂₋₁₈ fatty alcohols.

The amount and type of the further additive may depend on a number of factors, e.g. the HLB value of the surfactant. The weight ratio of the total amount of surfactants to the total amount of polymers is preferably from about 1:10 to 5:1, e.g. 1:10 to 1:3.

Preferably, however, no such additives are present or are only present in an amount less than 1% by weight based on the total weight of the composition.

The mono layer according to the invention may further contain skin penetration promoters, e.g. 1-dodecylazacycloheptan-2-one(azone) or N,N-diethyl-m-toluamide (DEET). If present, the weight ratio of skin penetration promoting agent to hydrophilic polymer is preferably from about 1:1 to 1:10. In a preferred embodiment of the invention however no skin penetration promotor is present or is only present in an amount less than 1% by weight of the mono layer.

The TTS of the invention can further comprise a backing layer and a release liner. The thickness of the monolayer is preferably in the range of 20 to 100µm, more preferably 60 to 100 µm.

The backing layer is preferably made of poly(ethylene terephthalate) PET foil. The backing layer should be thick enough to resist wrinkling which may arise upon prolonged periods in storage and by the movement of a subject's skin. Typically, the backing layer is, e.g. from approximately 10 µm to 100 µm, in thickness.

In a preferred embodiment, the backing layer is a double layer which consists of a PET layer as aforementioned and an EVA layer, e.g. Scotch Pack 1012 or to support transparent patches, a pure polyethylene terephthalate film.

In a very preferred embodiment of the invention, the backing layer comprises a laminate comprising a pigmented polyethylene, a thermoplastic resin and aluminum vapor coated polyester, e.g. Scotchpak 9738 Backing.

The release-liner may be a disposable element which serves to protect the pharmaceutical composition prior to its application. Typically the release-liner is produced from a material impermeable to the pharmaceutical and the adhesive. This release-liner may be easily stripped away from the pharmaceutical and/or adhesive. A preferred release-liner is made of poly(ethylene terephthalate) PET film, wherein the PET film is preferably a fluoropolymerized PET film and more preferably a siliconized PET film (e.g. Dreamliner). A release-liner, e.g. of about 50 to 250 µm, e.g. 100 µm thickness PET film, may be applied over the pharmaceutical composition.

The release liner may be silicone-coated. Said coating is preferably formed of any fluorosilicone compound which is conventionally used in the art, e.g a polyfluoroalkylsiloxane. It is particularly preferred to employ such a fluorosilicone coating when the adhesive used to affix the mono layer to the release liner is not itself a silicone adhesive.

TTS of the invention may be produced in a simple manner. A solvent-evaporation process may be used for producing said compositions. Thus all the ingredients of the composition which later forms the mono layer may be mixed in a solvent, e.g. acetone, ethylacetate or hexane, preferably ethylacetate, and cast onto a substrate which may act as the backing layer or the release-liner.

The mono layer may be conveniently formed in continuous sheets and may be cut into patches of any desirable size or configuration before use. However, the patches so-formed may expose the drug substance to the atmosphere, and therewith to oxidative stress. Hence in a preferred embodiment the TTS is manufactured under inert gas, e.g. nitrogen and/or the packaging devices use for the TTS, e.g. sachets, preferably are filled with inert gas, preferably with nitrogen. An inert gas as oft he invention is a gas/atmosphere containing less than 5%, preferred less than 1 % of oxygen. Hence in one embodiment the invention relates to a TTS which is packaged in a pouch in an atmosphere containing less than 5%, preferred less than 1 % of oxygen. Preferably the pouch is a four-seam pouch.

The pocket described hereinabove is preferably filled with an adhesive so as to encapsulate completely the discrete portion of the pharmaceutical composition. Preferably the adhesive is a silicone pressure sensitive adhesive as described hereinabove.

In a further embodiment the TTS of the invention the TTS is into a heat-sealable pouch comprising an acrylnitril-containing inner sealing layer. One preferred product for this layer is known as BAREX ™. In conjunction with the TTS of the invention improved stability had been recorded.

In a preferred embodiment the TTS of the invention is packaged in a four-seam pouch based on a laminate with an aluminium barrier laxer and a polymeric sealing layer containing polymer bound acrylnitrile.

It is an optional feature of all transdermal devices described hereinabove that they comprise a layer of adhesive between the pharmaceutical composition and the release liner.

The cover patch transdermal device may conveniently be formed as a continuous sheet or webbing and may be cut, or tom along a frangible area dividing each device, into patches before use although such devices may be provided as discrete patches.

The transdermal devices according to the invention are preferably packaged in multilayered packages, which contain an aluminium foil. Such packaging material is for example available as DanaExtra 15-06 or DanaExtra 15-07 by Danapak.

The transdermal devices of the invention in general have, for example an effective contact area of pharmaceutical composition on the skin of from about 1 to about 80, preferably form 2 to 50, square centimeters, in particular about 10 square centimeters, and are intended to be applied at intervals of about once every 1 to 7 days, preferably 1-3 days, in particular every day. Rivastigmine is well tolerated at a dose of 36 mg in free base form in up to 80 cm² of patches according to the invention containing 36 mg Rivastigmine from which 12 mg was absorbed. Rivastigmine may, for example, be administered at a dose of 8 mg in a patch of ca. 10 cm², once every day. The patch may be applied, for example on the abdomen, thigh, behind an ear, or on a shoulder or upper arm. A registered product (Exelon) even delivers 9.5 mg from a surface of 10 cm².

Due to its simple construction, TTS according to the invention have been shown to deliver the rate of 9.5 mg/d from a surface area of less then 8 cm², preferred 7 cm². Thus according to the invention a TTS is provided with preferably 7 cm² and a delivery rate of 9.5 mg/d. This allows patients to suffer less from wearing discomfort related to larger TTS.

The pharmaceutical compositions, optionally formed as a transdermal device, of the present invention are useful in particular for the treatment of Alzheimer's disease and

Parkinson related dementia. The exact amounts of Rivastigmine to be administered may depend on a number of factors, e.g. the drug release characteristics of the compositions, the drug penetration rate observed in vitro and in vivo tests, the duration of action required, the form of Rivastigmine, and for transdermal compositions the size of the skin contact area, and the part of the body to which the unit is fixed. The amount of and, e.g. area of the composition etc. may be determined by routine bioavailability tests comparing the blood levels of active agents after administration of Rivastigmine in a composition according to the invention to intact skin and blood levels of Rivastigmine observed after oral administration of a therapeutically effective dose of the compound.

A further subject of the present invention is the use of a pharmaceutical composition according to the invention or of a transdermal according to the invention for the treatment of a disease selected from Alzheimer's disease and Parkinson related dementia.

A further subject of the present invention is the use of a composition according to the invention for the manufacture of a medicament, wherein the medicament is preferably used for the treatment of Alzheimer's disease or Parkinson related dementia.

A further subject of the present invention is the matrix for a transdermal drug delivery system, comprising at least one poly alkyl acrylate and at least one poly alkyl methacrylate as hereinbefore defined, wherein the total amount of poly alkyl acrylate in the matrix is prerferably from 5 to 95 wt.-% and the total amount of poly alkyl methacrylate is also from 5 to 95 wt.-%.

The following examples illustrate the invention.

### Experimental

### Example 1: Compatibility test with different polymers

For finding a suitable matrix for stabilization of Rivastigmine base in absence of an antioxidant a compatibility test with a number of different adhesives was carried out. Besides poly alkyl acrylates (with and without functional groups) also polyisobutylene was tested. DuroTak 387-2353, which contains COOH groups, is the polymer which is used in the state of the art as matrix for Rivastigmine.

| Tab. 1: Tested adhesives | | | |
|---|---|---|---|
| Polymer | Type | Remark | Solvent |
| DuroTak 87-4098 | Acrylate-vinylacetate | No functional group | Ethyl acetate |
| DuroTak 87-9301 | Acrylate | No functional group | Ethyl acetate |
| DuroTak 387-2353 | Acrylate-vinylacetate | Functional group: -COOH | Ethyl acetate |
| DuroTak 387-2287 | Acrylate-vinylacetate | Functional group: -OH | Ethyl acetate |
| MG 0560 | Acrylate | Water-based acrylate adhesive | Water |
| DuroTak 87-618A | PIB | / | Hexane |

Whereas MG 0560 and DuroTak 87-618A were not suitable to form a stable matrix, all other polymers formed a stable matrix of Rivastigmine.

### Example 2: Stress test with different polymers

The matrices which showed sufficient stability were employed in a stress test to evaluate which of these polymers showed the best results concerning degradation of Rivastigmine. To do so, impurities were determined on day 0 as well as on day 6.

| Tab. 4: Stress stability of different adhesive systems | | | | | |
|---|---|---|---|---|---|
| Polymer | Time [Days] | Impurities [%] | | | |
| | | Imp 4 | RRT 0.44 | RRT 1.24 | Sum |
| DT 87-4098 | 0 | 0.08 | n.d. | 0.08 | 0.16 |
| | 6 | 0.28 | n.d. | 0.27 | 0.55 |
| DT 387-2287 | 0 | 0.10 | n.d. | 0.04 | 0.14 |
| | 6 | 0.27 | n.d. | 0.24 | 0.51 |
| DT 387-2353 | 0 | 0.13 | 0,07 | n.d. | 0.20 |
| | 6 | 0.84 | n.d. | 0.49 | 1.33 |
| BIO-PSA 7-4202 | 0 | 0.07 | n.d. | n.d. | 0.07 |
| | 6 | 0.20 | n.d. | 0.17 | 0.37 |
| DT 87-9301 | 0 | n.d. | n.d. | n.d. | n.d. |
| | 6 | 0.15 | n.d. | 0.15 | 0.30 |

It turned out, that whereas DT 387-2353 which contains free carboxylate groups and which is the polymer that is used in the state of the art for stabilizing Rivastagmine, shows only pour stability, all other polymers, i.e. the poly alkyl acrylate polymers with no free carboxylate groups, show good stability results for Rivastagmine.

### Example 3: Comparison of in vitro and in vivo peel strength

The peel strength is the force, measured in Newton, that must be exerted to remove the patch with a defined velocity from a stainless steel test plate.

### a) Equipment and materials

Tensile testing device (e.g. Zwick BT1-FR2.5TN.D14) including PC/application
Adequate load cell (e.g. 100 N)
Grips enabling measurement in 90 ° angle
Peeling apparatus (Sliding table), for measuring at 90 ° angle
Stainless steel test plate eg. V4A-AISI 316L
Separation aid: adhesive tape (e.g. tesafix 4963)
Cleaning agent: acetone
Die cut and die cut form (25 mm width)

### b) Sample preparation

25 mm wide stripes are die cutted out of the patches, which are equilibrated to the climatic measuring conditions (23°C ± 3°C / 50% ±10% r.h.) for at least 2 h prior to measurement.

### c) Test procedure

The 100 N load cell and the sliding table including the stainless steel testing plate are installed. The test plate is cleaned with acetone.

After the separation aid is affixed to the patch, the release liner is removed completely from the patch and the patch is adhered to the stainless steel plate without pressure, air bubbles or wrinkles. The separation aid is then fixed into the clamp of the tensile testing device in such a way that the patch is peeled off of the test plate in a 90° angle running a test velocity of 300 mm/min. The measurement has to be carried out in a timeframe of 30 to 60 seconds after adhering the sample to the test plate.

### d) Evaluation of the data

The standard procedure comprises 6 samples. The average value among the mean forces occurred within the measurement range is reported as N /25 mm.

Rivastigmine is a pharmaceutical which is liquid at room temperature. This characteristic influences the physical properties of the patch, resulting in a very soft adhesive system. In addition, the relative high Rivastigmine content in the patch had a direct influence on the adhesive properties of the patch adhesion (*in vitro* and *in vivo*). Prototypes composed on basis of the acrylate polymers exhibited good physical stability. To optimise the adhesion force the formulation was modified by usage of non-adhesive methacrylate polymers (Eudragit EP O and Plastoid B) which are also free of free carboxylate groups. *In vivo* adhesion testing required the development of a placebo patch with the same physical properties. Because of the high influence of Rivastigmine on the physical properties a substitute was used to simulate Rivastigmine in the placebo patch. Different prototypes were tested and compared to the verum formulations using *in vitro* adhesion testing. A suitable placebo formulation could be deduced and was tested *in vivo* using a human test panel. Suitable adhesion could be shown over a time period of 24 hours.

### Example 4: Prototypes

Based on the compatibility, stability and peel strength experiments, two prototype patches were developed, which contained the poly alkyl acrylate DT 87-9301 and a poly alkyl methacrylate selected from Plastoid B and Eudragit E PO, as can be seen in the following table.

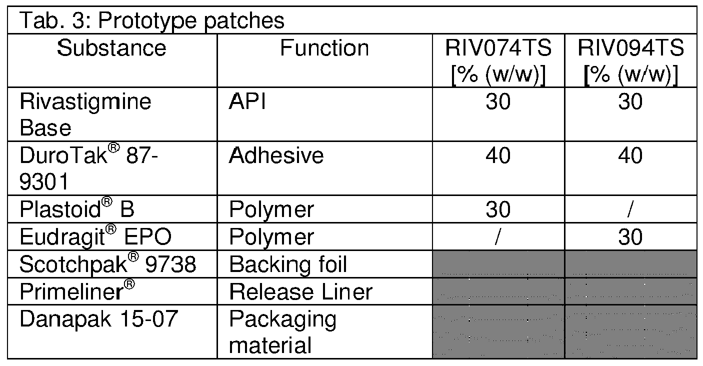

### Example 5: In vitro skin permeation testing with the prototypes

Comparison of the skin permeation between the product according to the state of the art and the prototypes was performed using a Franz cell *in vitro* model based on dermatomised human skin.

The *in vitro* skin permeation testing was carried out as follows:
The test sample (1.5 cm²) was placed on the skin and pressed on the skin for a short time (10 s). The patches adhered to skin were applied on the Franz Cell which is placed in the water bath for the permeation. At each sampling time the Franz Cell is removed from the water bath. A 2 ml sample of the acceptor medium (PSB, pH 5) is withdrawn and aliquots are transferred into HPLC vials for analysis. After that 2 ml of the acceptor medium are replaced to the cell and the cell is replaced to the bath. After each sampling time, the samples are stored at 2-8°C in the refrigerator until analysis.

All prototypes tested performed similar to the product according to the state of the art with regard to the permeation kinetic. The flux in the linear phase of the prototypes was even higher compared to the product according to the state of the art, indicating a potential for a reduced patch size.

### Example 6: Stability testing with the prototype

Stability testing was performed under ICH longterm and accelerated conditions over a time period of 6 months. Thresholds of impurities were calculated using ICH Topic Q 3 B (R2) "Impurities in New Drug Products". Based on maximal daily dose of an 9.5 mg/24 h patch the reporting threshold (RT) was 0.1%, the identification threshold (IT) was 0.5% and the qualification threshold (QT) was 0.53%.

The results (Tab. 4) suggested that the formulation was stable at 25 °C according to the set limit of 0.53% representing the qualification threshold for known impurities and 0.5% for unknown impurities. After 6 months at 40 °C impurity 4 showed a borderline result to the identification/qualification threshold. Those results suggested a storage advice of "do not store above 25 °C".

Assay decreased over time faster at 40 °C compared to 25 °C. It could be deduced that the API migrated into the packaging material. After switching the packaging material of the prototypes from Surlyn^{®} to Barex^{®} the migration effect could be inhibited.

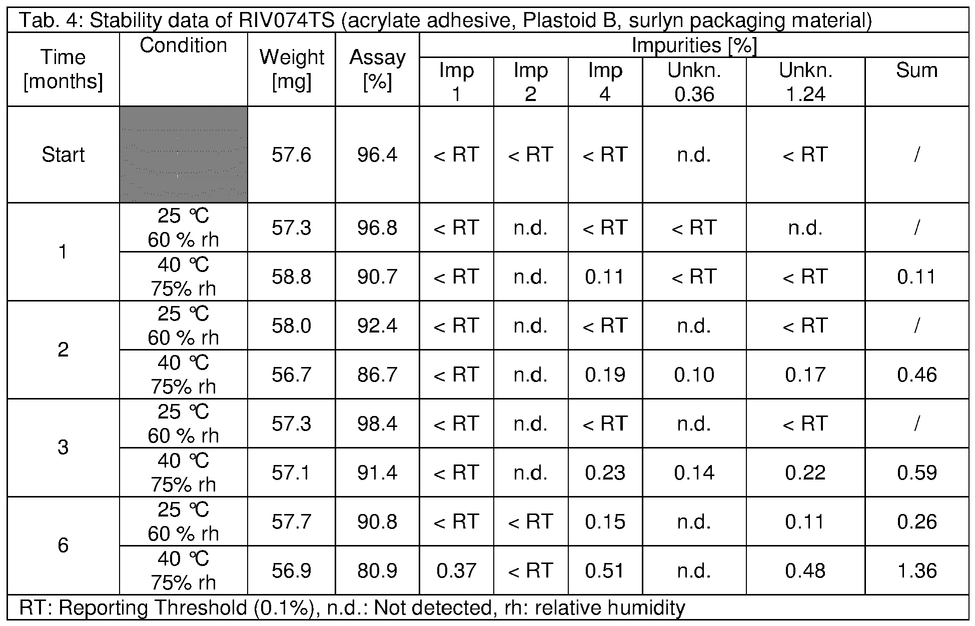

### Example 7: Clinical testing of the TTS of the invention

In a clinical study the rate and extent of absorption of a reference Rivastigmine TTS (the product marketed under the brand "Exelon") with 9.5mg/24h and the TTS of the invention was compared. The TTS were administered as a single dose of 1 x 9.5mg/24hr to healthy subjects under fasting conditions. The study was established as a single center study, randomized, 2-way crossover under fasting conditions. 12 healthy adult males and females (18 years old and older), non-smokers, were included. A single dose (1 x 9.5mg/24hr) was worn for 24 hrs in each period with a washout of 3 days or more between doses.

A total of 15 blood samples was taken per period: at pre-dose and at 2, 4, 6, 8, 10, 12, 16, 20, 24, 26, 28, 30, 32 and 36 hours post-dose.

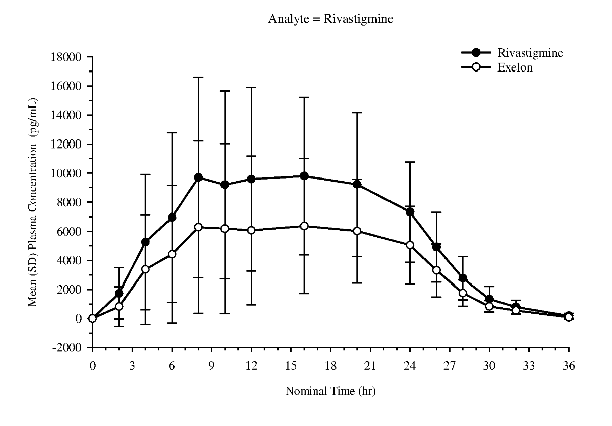

The mean plasma concentration of the API is depicted in Fig. 1.

## Claims

1. A transdermal therapeutic system (TTS) with a monolayer comprising at least one phenyl carbamate, at least one poly alkyl acrylate and at least one poly alkyl methacrylate, **wherein** the poly alkyl acrylate and the poly alkyl methacrylate are substantially free of free carboxylate groups.

2. A transdermal therapeutic system according to claim 1, **wherein** the phenyl carbamate is (*S*)-{3-[α-(Dimethylamino)ethyl]phenyl}-N-ethyl-N-methylcarbamate (Rivastigmine).

3. A transdermal therapeutic system according to claim 1 or 2, which constitutes a monolithic TTS, preferably with at 10% wt-%, preferably at least 25 wt-% Rivastigmine.

4. A transdermal therapeutic system according to any of the preceding claims, **wherein** the poly alkyl acrylate and the poly acryl methacrylate form a biphasic system.

5. A transdermal therapeutic system according to claim 4, **wherein** the poly alkyl methacrylate constitutes inner phase and the poly alkyl acrylate constitutes the continuous phase of the biphasic system.

6. A transdermal therapeutic system according to any of the preceding claim, comprising
a) 1 to 60 wt.-% of a phenyl carbamate, in particular Rivastigmine;
b) 5 to 70 wt.-% of at least one poly alkyl acrylate which is substantially free of free carboxylate groups; and
c) 5 to 70 wt.-% of at least one poly alkyl methacrylate which is substantially free of free carboxylate groups.

7. A transdermal therapeutic system according to any of the preceding claims, **wherein** the poly alkyl acrylate and the poly alkyl methacrylate are **characterized by** having no free carboxylate group.

8. A transdermal therapeutic system according to any of the preceding claims, **wherein** the poly alkyl acrylate and the poly alkyl methacrylate are **characterized by** comprising C₁₋₁₀ alkyl ester groups, more preferably C₁₋₆ alkyl ester groups.

9. A transdermal therapeutic system according to any of claims 1 to 8, **wherein** the poly alkyl methacrylate is **characterized by** comprising heteroalkyl ester groups, preferably C₁₋₁₀ heteroalkyl ester groups, more preferably C₁₋₆ heteroalkyl ester groups, preferably selected from hydroxy alkyl ester groups, preferably hydroxy C₁₋₁₀ alkyl ester groups, more preferably hydroxy C₁₋₆ alkyl ester groups, and/or from heteroalkyl ester groups which contain nitrogen, in particular in the form of a tertiary amino group.

10. A transdermal therapeutic system according to any of the preceding claims, **wherein** the poly acrylate is selected from the group consisting of
a) homopolymers of alkyl acrylate monomers;
b) copolymers of alkyl acrylate monomers and vinyl acetate monomers;
c) copolymers of alkyl acrylate monomers and nitrogen containing non-ionic monomers, wherein the nitrogen containing monomers are preferably selected from N-substituted acrylamide monomers, N-substituted methacrylamide monomers, vinylacetamides, nitriles and mixtures thereof and preferably present in an amount of 2-50 %.

11. A transdermal therapeutic system according to any of the preceding claims, **wherein** the poly methacrylate is a homopolymer of methacrylate monomers and optionally contains heteroalkyl ester groups which contain nitrogen in the form of tertiary amino groups.

12. A transdermal therapeutic system according to any of the preceding claims, **wherein** the poly methacrylate contains dimethylaminoethyl methacrylate, preferably at least 0,5% (g/g).

13. A transdermal therapeutic system according to any of the preceding claims, **characterized by** containing no poly (meth)acrylate, preferably no polymer at all, more preferably no compound at all, with a free carboxylate group.

14. A transdermal therapeutic system according to any of the preceding claims, **wherein** the transdermal therapeutic system does not contain any antioxidant.

15. A transdermal therapeutic system according to any of the preceding claims, **wherein** the drug delivery rate in humans exceeds 1.2 mg/24hrs.cm², preferred exceeds 1.4 mg/24hrs.cm².

16. A transdermal therapeutic system according to any of the preceding claims, wherein the size if the transdermal therapeutic system preferably delivering 9.5 mg/d is below 10 square cm, preferably below 8 square cm, most preferred 7 square cm.
